# EUROPEAN PATENT SPECIFICATION

(11) **EP 0 488 424 B1**
(45) Date of publication and mention of the grant of the patent: **05.03.1997**
(21) Application number: 91120685.2
(22) Date of filing: 02.12.1991
(51) Int. Cl.: C12N 15/60, C12N 15/61, C12P 13/22

(54) **Recombinant DNA sequences encoding feedback inhibition released enzymes, plasmids comprising the recombinant DNA sequences, transformed microorganisms useful in the production of aromatic amino acids, and a process for preparing aromatic amino acids by fermentation**
Rekombinante DNS-Sequenzen kodierend für Enzyme frei von Feedback Inhibition, Plasmide diese Sequenzen enthaltend, transformierte Mikroorganismen nützlich für Produktion von aromatischen Aminosäuren, und deren Verfahren zur Herstellung durch Fermentation
Séquences d'ADN recombinantes codant pour des enzymes insensibles à l'inhibition de feedback, plasmides les comprenant, micro-organismes transformés utiles à la production d'acides aminés aromatiques, et procédé pour les préparer par fermentation

(30) Priority: 30.11.1990 JP 337221/90; 02.05.1991 JP 196226/91
(43) Date of publication of application: 03.06.1992
(60) Divisional application: 96105800.5
(73) Proprietor: Ajinomoto Co., Inc., Tokyo 104 (JP)
(72) Inventor: Tonouchi, Naoto, Central Res.Lab.Ajinomoto Co.Inc., Kawasaki-shi, Kanagawa-ken (JP); Kojima, Hiroyuki, Central Res.Lab.Ajinomoto Co.Inc, Kawasaki-shi, Kanagawa-ken (JP); Matsui, Hiroshi, Central Res.Lab.Ajinomoto Co.Inc., Kawasaki-shi, Kanagawa-ken (JP)
(74) Representative: Strehl Schübel-Hopf Groening & Partner

(56) References cited:
- EP-A- 0 264 914
- EP-A- 0 330 773
- FR-A- 2 486 961
- Week 8701, Derwent Publications Ltd., London, GB; AN 87-002443 [01] & JP-A-61 260 892 (KYOWA HAKKO KOGYO KK) 19 November 1986
- JOURNAL OF BACTERIOLOGY, vol.172, no.11, 1990, BALTIMORE US, pages 6581-6584; Weaver, Lisa M. & Herrmann, Klaus M.: 'Cloning of an aroF allele encoding a tyrosine-insensitive 3-deoxy-D-arabino-heptulosonate 7-phosphate synthase'
- JOURNAL OF BACTERIOLOGY, vol.170, no.12, 1988, BALTIMORE US, pages, 5500-5506; Ray, Jill M.; Yanofsky, Charles; Bauerle, Ronald: 'Mutational analysis of the catalytic and feedback sites of the tryptophan-sensitive 3-deoxy-D-arabino-hep tulosonate-7-phosphate synthase of Escherichia coli'

## Description

### BACKGROUND OF THE INVENTION

### Field of the Invention:

The present invention relates to recombinant DNA sequences encoding feedback inhibition released enzymes, plasmids containing these recombinant DNA sequences, microorganisms transformed with these plasmids, and a process for preparing L-tryptophan, L-phenylalanine and L-tyrosine by fermentation.

### Discussion of the Background:

Demand for aromatic amino acids is rapidly increasing. For example, L-phenylalanine is used as a raw material for the sweetener aspartame, L-tryptophan is an important feed additive, and all three (L-phenylalanine, L-tryptophan, and L-tyrosine) are useful as transfusion drugs.

Many methods for preparing aromatic amino acids using microorganisms are known. For example, methods for preparing L-phenylalanine using recombinant Escherichia coli are described in Japanese Published Unexamined Patent Application Nos. 56-1890, 58-103398, 61-92565 and 1-104160, and World Patent Publication WO 87/00202. A method for preparing L-phenylalanine or L-tyrosine using a mutant belonging to Coryneform bacteria is described in Japanese Patent Published Unexamined Application No. 61-128897, and methods using recombinant Coryneform bacteria are described in Japanese Unexamined Published Patent Application Nos. 60-34197, 60-24192, 61-260892 and 61-124375. A method for preparing L-tryptophan using recombinant E. coli is described in Japanese Published Unexamined Patent Application No. 57-71397 and U.S. Patent No. 4,371,614; methods using mutants of Bacillus subtilis are described in Japanese Published Unexamined Patent Application Nos. 53-39517 and 62-34399; methods using recombinant Bacillus subtilis are described in Japanese Patent Published Unexamined Application Nos. 61-104790 and 62-34399; methods using a mutant of Coryneform bacteria are described in Japanese Published Unexamined Patent Application No. 57-174096; and a method using recombinant Coryneform bacteria is described in Japanese Published Unexamined Patent Application No. 62-51980.

Generally, in the biosynthetic route of aromatic amino acids, a key enzyme which plays a central role in the biosynthesis is subject to feedback inhibition by the final product. In the methods described above, the desired amino acids are principally produced using microorganisms wherein the key enzyme is released from feedback inhibition by the final product. The key enzymes released from feedback inhibition in the above methods include 3-deoxy-D-arabinoheptulonic acid-7-phosphate synthase (hereafter abbreviated as "DS") and prephenate dehydratase (hereafter abbreviated as "PD").

Turning first to DS, among the microorganisms used in the methods described above, Escherichia coli has three types of naturally-occurring (wild-type) DS isozymes. These isozymes are encoded by genes called aroF, aroG and aroH, which are subject to feedback inhibition by L-tyrosine, L-phenylalanine and L-tryptophan, respectively.

The nucleotide sequences and amino acid sequences relevant to these genes and enzymes are already reported [aroF: Hudson, G.S. and Davidson, B.E., J. Mol. Biol., 180, 1023 (1984); aroG: Davies, W.D. and Davidson, B.E., Nucleic Acids Res., 13, 4045 (1982); aroH: Ray, J.M. et al., J. Bacteriol., 170, 5500 (1988)].

In order to efficiently produce the desired aromatic amino acids, expression of these DS genes must be improved. With respect to aroH-encoded DS, feedback inhibition by L-tryptophan has been released using mutant aroH [Ray, J.M. et al., J. Bacteriol., 170, 5500 (1988)]. However, the DS activity derived from aroH is very poor, and the aroH-derived DS is unsuitable for improvement by recombinant DNA techniques. It is more efficient to utilize aroF- or aroG-encoded DS in which feedback inhibition is released ("feedback inhibition-released" DS).

An example of a mutation which releases feedback inhibition of aroF-encoded DS by L-tyrosine is the substitution of the 148 proline residue from the N-terminus (¹⁴⁸Pro) with a leucine residue [Weaver, L.M. and Herrmann, K.M., J. Bacteriol., 172, 6581 (1980)].

Only a few examples as shown below for the production of aromatic amino acids by fermentation employ feedback inhibition-released DS with a clearly shown mutation site. Edwards et al. teach that feedback inhibition by L-tyrosine in the production of L-phenylalanine by fermentation is released by substituting the 152 glutamine residue (¹⁵²Gln) of DS encoded by aroF with isoleucine [WO 87/00202]. Furthermore, Sinenki et al. teach that feedback inhibition by L-phenylalanine in the production of L-phenylalanine by fermentation is suppressed by substituting the 76 leucine residue (⁷⁶Leu) of DS encoded by aroG with valine [Japanese Published Unexamined Patent Application No. 58-103398]. However, the enzyme activity of the feedback inhibition-released DS and the amount of L-phenylalanine produced are unknown. No reports of the production of L-tryptophan by feedback inhibition-released DS mutants are known.

Turning next to PD, a wild-type bifunctional enzyme (CM-PD) present in Esherichia coli having both chorismate mutase (hereafter abbreviated as "CM") activity and PD activity is subject to feedback inhibition by L-phenylalanine. The enzyme is encoded by a gene called pheA. The nucleotide sequence of pheA and the amino acid sequence of wild-type CM-PD are known [Hudson, G.S. and Davidson, B.E., J. Mol. Biol., 180, 1023 (1984)]. In order to efficiently produce L-phenylalanine, it is important to release the feedback inhibition of CM-PD by L-phenylalanine.

Some examples of modification and mutation on an amino acid level are known to release feedback inhibition for the fermentative production of L-phenylalanine. By modifying two tryptophan residues (226 and 338 amino acids from the Nterminus) of CM-PD with dimethyl(2-hydroxy-5-nitrobenzylsulfonium bromide), an enzyme having resistance to feedback inhibition can be obtained [Gething, M.J.H, and Davidson, B.E., Eur. J. Biochem., 78, 111 (1977)]. Feedback inhibition-released enzyme can be obtained by deleting the 338 tryptophan residue (³³⁸Trp) or substituting ³³⁸Trp and the subsequent residues with arginine-glycine (Japanese Published Unexamined Patent Application. No. 1-235597). Inserting the amino acid sequence tryptophan-arginine-serine-proline into the site of the same 338 tryptophan residue also releases feedback inhibition (WO 87/00202). These techniques focus on the 338 tryptophan residue. However, no definitive study on the effects of modifying. or mutating the ²²⁶Trp residue has been performed.

On the other hand, in Coryneform bacteria, PD is subject to feedback inhibition by L-phenylalanine. A gene in which the feedback inhibition by L-phenylalanine has been released is known. [Ozaki, A. et al., Agric. Biol. Chem., 49, 2925 (1986); Ito, H. et al., Appl. Microbiol. Biotechnol., 33, 190 (1989)]. The nucleotide sequence of the wild type Coryneform PD gene shows homology to the pheA gene of Escherichia coli K-12 [Follettie, M.T. and Sinsky, A.J., J. Bacteriol., 167, 695 (1986)]. However, the nucleotide sequence of the feedback inhibition-released PD gene in Coryneform bacteria is unknown, as is mutation of the nucleotide sequence and release of feedback inhibition by substitution of the corresponding amino acid sequence.

### SUMMARY OF THE INVENTION

Accordingly, one object of the present invention is to provide a process for efficiently preparing an aromatic amino acids by fermentation.

A further object is to provide transformed microorganisms useful in the production of aromatic amino acids by fermentation.

A further object is to provide recombinant plasmids which express genes encoding key enzymes in the biosynthesis of aromatic amino acids in which feedback inhibition is released.

A further object is to provide recombinant DNA sequences which encode key enzymes in the biosynthesis of aromatic amino acids in which feedback inhibition is released.

A further object is to provide novel recombinant enzymes which are important in the biosynthesis of aromatic amino acids in which feedback inhibition is released.

These and other objects which will become apparent during the following detailed description of the preferred embodiments have been accomplished by a recombinant DNA sequence encoding an enzyme of the aromatic amino acid biosynthetic pathway, wherein feedback inhibition is released, a plasmid comprising the recombinant DNA sequence, a microorganism useful in the production of aromatic amino acids transformed with one or more of the plasmids, and a process for preparing an aromatic amino acid which comprises culturing the transformed microorganism and isolating the aromatic amino acid produced thereby.

The above objects of the present invention are accomplished by a DNA fragment encoding chorismate mutase-prephenate dehydratase from Escherichia coli, in which the serine residue in position 330 is substituted or amino acid residues downstream from the 330 serine residue are deleted, and a DNA fragment encoding prephenate-dehydratase from Coryneform bacterium in which the serine residue in position 235 is substituted.

Another embodiment of the present invention are vectors and microorganisms containing and expressing said DNA fragments.

A further embodiment of the present invention is a microorganism which, in addition to a mutated gene encoding PD or CM-PD, comprises a mutated gene encoding 3-deoxy-D-arabinoheptulonic acid-7-phosphate synthase derived from the genus Escherichia, wherein one or two amino acid residue(s) are substituted or one or more amino acid residue(s) are deleted, and feedback inhibition is released.

According to the present invention, microorganisms producing mutated PD or CM-PD alone or together with feedback inhibition-released DS were used to prepare aromatic amino acids.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
Fig. 1 shows the construction of the plasmids pTS-aroF and pTS-aroG;
Fig. 2 shows the extent of inhibition by L-tyrosine of activity in DS encoded by both wild-type and mutant aroF;
Fig. 3 shows the extent of inhibition by L-phenylalanine of activity in DS encoded by both wild type and mutant aroG.
Fig. 4 shows the extent of inhibition by L-phenylalanine in the prephenate dehydratase activity of both wild-type and mutant chorismate mutase-prephenate dehydratase;
Fig. 5 shows the construction of the plasmid pACKG4.

### DETAILED DESCRIPTION OF THE PREFERRED EMBODIMENTS

The present invention relates to a mutant feedback inhibition-released enzyme in the biosynthetic pathway of aromatic amino acids; a recombinant DNA sequence encoding an enzyme of the aromatic amino acid biosynthetic pathway, wherein feedback inhibition is released; a plasmid comprising a recombinant DNA sequence encoding an enzyme of the aromatic amino acid biosynthetic pathway, wherein feedback inhibition is released; a microorganism useful in the production of aromatic amino acids transformed with one or more plasmids comprising a recombinant DNA sequence encoding an enzyme of the aromatic amino acid biosynthetic pathway, wherein feedback inhibition is released; and a process for preparing an aromatic amino acid which comprises culturing a microorganism transformed with one or more plasmids comprising a recombinant DNA sequence encoding an enzyme of the aromatic amino acid biosynthetic pathway, wherein feedback inhibition is released, and isolating the aromatic amino acid produced thereby.

In the present application, the phrase "aromatic amino acid" refers to L-phenylalanine, L-tryptophan and L-tyrosine. Also, an enzyme is "released" from feedback inhibition by a final product if the activity of the enzyme doesn't decrease in the presence of the final product.

Preferably, the enzymes of the present invention in the biosynthetic pathway of aromatic amino acids which are to be released from feedback inhibition are 3-deoxy-D-arabinoheptulonic acid-7-phosphate synthase (DS), prephenate dehydratase (PD) and chorismate mutase-prephenate dehydratase (CM-PD). The means by which each of these enzymes is released from feedback inhibition in the present invention is preferably by mutation, wherein one or two amino acid residue(s) are substituted with other amino acid residue(s) or one or more amino acid residue(s) are deleted. Furthermore, the transformed microorganism preferably belongs to the genus Escherichia, and is preferably transformed with one or more plasmids bearing a recombinant DNA sequence corresponding to one of the above mutant enzymes.

Firstly, the present Inventors have acquired a gene encoding DS, wherein the feedback inhibition is released by cloning the natural DS gene of Escherichia coli and subjecting the cloned gene to mutation. Furthermore, the natural PD gene of Brevibacterium lactofermentum is cloned, and a gene encoding PD in which feedback inhibition is released, is cloned from L-phenylalanine-producing Coryneform bacteria. Even further, the natural CM-PD gene of Escherichia coli is cloned, and the cloned gene is then subjected to mutation to produce a novel gene encoding CM-PD in which feedback inhibition is released. By transfecting or transforming phenylalanine-producing bacteria with mutated genes of PD or CM-PD alone or together with a gene encoding feedback inhibition-released DS production of L-phenylalanine by fermentation can be improved.

The gene encoding DS having released feedback inhibition is prepared by the following procedure.

Firstly, aroF and aroG genes are cloned from chromosomal DNA of Escherichia coli using the PCR method as described in U.S. Patent Nos. 4,800,159, 4,683,202 and 4,683,195, all incorporated herein by reference. The chromosomal DNA appropriate as a source of the aroF and aroG genes for use in the present invention may be cloned from any strain of Escherichia coli, but the preferred strain is K-12 MC1061 (ATCC 53338). The desired genes are then mutated with hydroxylamine by a known method; for example, that described in J. Mol. Biol., 175, 331 (1984).

The genes aroF and aroG encode DS which is subject to feedback inhibition by L-tyrosine and L-phenylalanine, respectively, and also include mutants caused by genetic polymorphism, etc. Genetic polymorphism refers to a modification of an amino acid sequence of a protein due to natural mutation of a gene.

In order to cause mutation of the gene, a number of effective methods are known. Examples include recombinant PCR methods [PCR Technology, Stockton Press (1989)], site specific mutation [Kramer, W. and Frits, H.J., Methods in Enzymology, 154, 350 (1987)], conventional methods exposing a strain bearing the gene to UV (ultraviolet light) rays, conventional methods treating the DNA or DNA-bearing microorganism with a chemical (N-methyl-N'-nitrosoguanidine, nitric acid, etc.), and conventional methods for chemical synthesis of the desired gene, such as those employing a known automated synthesizer.

In the present invention, the mutated amino acid residue of DS is in the region of the amino acid sequence which participates in the mechanism of feedback inhibition by L-tyrosine, L-phenylalanine or L-tryptophan. For example, in DS encoded by aroF, the 147 aspartic acid residue (¹⁴⁷Asp) and the 181 serine residue (¹⁸¹Ser) from the N-terminus are the mutated amino acid residues. Any mutation of the amino acid residue which results in release from feedback inhibition is suitable. For example, substitution, deletion, or addition is suitable. The DS mutations and the corresponding nucleotide sequence mutations exemplified in the present invention are summarized in Table 1.

By transfecting a suitable microorganism with the mutant aroF or aroG gene above as a recombinant DNA sequence, the microorganism can express the recombinant mutant gene in which feedback inhibition is released.

In the present invention, the novel gene encoding PD in Brevibacterium lactofermentum and the novel gene encoding CM-PD in Escherichia coli were prepared as follows.

Firstly, the nucleotide sequence of the Brevibacterium lactofermentum PD gene encoding PD in which feedback inhibition by L-phenylalanine is released was determined and analyzed. It has thus been found that the L-phenylalanine-producing strain expressed PD in which one amino acid is substituted, as compared to the wild strain. Next, based on this finding, a substitution or a deletion of amino acid residue(s) was carried out at the corresponding position of CM-PD in Escherichia coli K-12, resulting in CM-PD in which the feedback inhibition is released.

Enzymes having PD activity referred to in the present invention refer to enzymes derived from microorganisms such as Coryneform bacteria having PD activity, and further refer to enzymes derived from microorganisms such as Escherichia coli, etc., having the bifunctional activity of CM-PD.

In the present invention, the mutated amino acid residue of PD refers to a substitution of an amino acid residue or a deletion of amino acid residue(s) present in the region of the amino acid sequence which participates in the mechanism of feedback inhibition by L-phenylalanine. For example, in PD derived from Brevibacterium lactofermentum, the 235 serine residue (²³⁵Ser) is suitable for mutation, and in CM-PD derived from Escherichia coli, the 330 serine residue (³³⁰Ser) from the N-terminus is an amino acid residue suitable for mutation. Suitable mutations include any which result in the release of feedback inhibition, but particularly suitable mutations include substitutions of ²³⁵Ser or ³³⁰Ser with proline or aspartic acid residue, or deletion of amino acid residues down stream from ³³⁰Ser.

By transfecting a suitable microorganism with the mutant PD or CM-PD gene described above as a recombinant DNA sequence, expression of PD in which feedback inhibition is released is achieved in the transfected microorganism.

The recombinant DNA sequences obtained by the foregoing procedures refer to those obtained by incorporating a gene encoding feedback inhibition-released DS or PD into a vector of plasmid or phage DNA. In the present invention, promoters such as lac, trp, PL, etc. which act in the microorganism may also be used to efficiently perform the expression of the gene. The recombinant DNA sequences referred to herein include those obtained by incorporating one or more of the above-described genes into a chromosome according to known methods. Examples include methods using a transposon (Berg, D.E. and Berg, C.M., Bio/Technol., 1, 417 (1983)), Mu phage (Japanese Published Unexamined Patent Application No. 2-109985) or homologous recombination [Experiments in Molecular Genetics, Cold Spring Harbor Laboratory (1972)].

As the microorganism containing the recombinant DNA, any microorganism may be used, irrespective of species and strain of the microorganism, so long as it expresses the gene encoding the desired enzyme (such as DS or PD) and is capable of producing the aromatic amino acid (for example, in the case of L-phenylalanine, the microorganism which has acquired L-phenylalanine productivity by imparting L-phenylalanine analog resistance thereto). Particularly suitable microorganisms are selected from the genus Escherichia, the genus Brevibacterium, the genus Corynebacterium, the genus Bacillus, the genus Serratia, the genus Pseudomonas, etc.

The thus obtained microorganism transformed by the recombinant DNA bearing the PD gene or a microorganism additionally producing the feedback inhibition-released DS is cultured, the desired aromatic amino acid is produced by the transformed microorganism in a suitable medium, and the accumulated aromatic amino acid is collected and isolated.

The medium used for producing the aromatic amino acid is a conventional medium containing appropriate carbon sources, nitrogen sources, inorganic ions and, if necessary, other organic components.

Suitable carbon sources include sugars such as glucose, lactose, galactose, fructose, starch hydrolysate, etc.; alcohols such as glycerol, sorbitol, etc.; organic acids such as fumaric acid, citric-acid, succinic acid, etc.

Suitable nitrogen sources include inorganic ammonium salts such as ammonium sulfate, ammonium chloride, ammonium phosphate, etc.; organic nitrogen such as soybean hydrolysate, etc.; ammonia gas, ammonia water, etc.

Suitable organic trace nutrient sources preferably are present, and include required substances such as vitamin B₁, L-tyrosine, or yeast extract, etc., in an appropriate amount.

In addition thereto, small amounts of potassium phosphate, magnesium sulfate, iron ions, manganese ions, etc. may be present.

Incubation is carried out for 16 to 72 hours under aerobic conditions. The temperature for incubation is maintained between 30 and 45°C and the pH is maintained in the range of 5 to 7 during the incubation. The pH may be adjusted with either acids or alkaline substances, which may be inorganic or organic, or may be adjusted with ammonia gas, etc., as is appropriate to maintain the desired pH and concentrations of components in the medium.

The desired aromatic amino acid is isolated from the fermentation medium generally by conventional methods, such as use of an appropriate ion exchange resin, precipitation, and/or other known techniques, either alone or in combination.

By the general process described above, the transformant expressing feedback inhibition-released PD or CM-PD alone or together with feedback inhibition-released DS is obtained, and by culturing the transformant, the productivity of aromatic amino acids can be greatly improved.

Other features of the invention will become apparent in the course of the following descriptions of exemplary embodiments which are given for illustration of the invention, and are not intended to be limiting thereof.

### Example 1: Preparation of a gene encoding DS in which the feedback inhibition is released

### (1) Collection of an aroF-derived mutant DS gene of Escherichia coli

Chromosomal DNA was extracted from Escherichia coli K-12 MC1061 strain in a conventional manner. In a separate procedure, two synthetic DNA primers shown by Sequence Nos. 1 (SEQ ID NO:1) and 2 (SEQ ID NO:2) were synthesized in a conventional manner, based on the known nucleotide sequence of the target aroF gene [J. Mol. Biol., 180, 1023 (1984)].

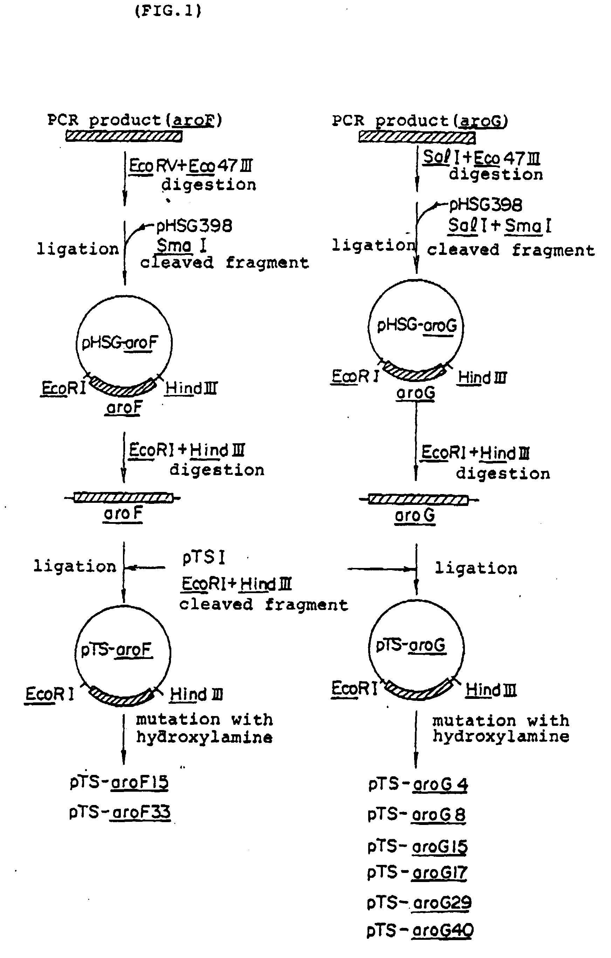

These primers have homologous sequences upstream and downstream from the aroF gene. Using the chromosomal DNA and the DNA primers, PCR (polymerase chain reaction) is conducted according to the method of Erlich et al. [PCR Technology, Stockton Press (1989)], yielding a DNA fragment of 1.5 Kbp. Thereafter, as shown in Fig. 1, left side, the fragment is cleaved with restriction enzymes EcoRV and Eco47III, and the product is then ligated with the SmaI digestion product of pHSG398 (manufactured by Takara Shuzo) using T4 DNA ligase. Competent cells of Escherichia coli JM109 strain (manufactured by Takara Shuzo) were transformed with the reaction mixture. A plasmid having the aroF gene was extracted from the strains resistant to chloramphenicol to yield the plasmid pHSG-aroF.

Subsequently, pHSG-aroF was digested with restriction enzymes EcoRI and HindIII, and the resulting DNA fragment bearing the aroF gene was ligated with the EcoRI and HindIII digestion fragment of plasmid pTS1 (Japanese Patent Application No. 2-192162) using T4 DNA ligase. Competent cells of DS-deleted (aroF, aroG, aroH) strain AB3257 of Escherichia coli K-12 were transformed with the reaction mixture (AB3257 strain was acquired from the Escherichia coli Genetic Stock Center). From among the strains resistant to ampicillin, the strain in which auxotrophy of L-tyrosine, L-phenylalanine and L-tryptophan disappeared was selected, and a plasmid was extracted therefrom, yielding plasmid pTS-aroF.

Next, after mutation of plasmid pTS-aroF using hydroxylamine according to the method of J. Mol. Biol., 175, 331 (1984), the mutant was used to transform the E. coli AB3257 strain. After ampicillin-resistant strains were collected, two strains which grew in minimum medium supplemented with 1 mM L-tyrosine were selected. From these strains, plasmids pTS-aroF15 and pTS-aroF33 bearing the genes encoding feedback inhibition-released DS were obtained.

Cells of AB3257 strain transformed with plasmids containing the gene encoding non-feedback inhibition-released DS are subject to feedback inhibition at 1 mM concentration of L-tyrosine in the minimum medium. Accordingly, the strain subject to feedback inhibition failed to synthesize aromatic amino acids such as L-phenylalanine or L-tryptophan, and failed to grow.

### (2) Preparation of aroG-derived mutant DS gene of Escherichia coli

A mutant aroG gene was collected in a manner similar to the case of the aroF gene. Two synthetic DNA primers shown by Sequence Nos. 3 (SEQ ID NO:3) and 4 (SEQ ID NO:4) were synthesized in a conventional manner, based on the known nucleotide sequence of the aroG gene (Nucleic Acids Res., 10, 4045 (1982)).

Using the primers and the chromosomal DNA of the E. coli MC1061 strain, PCR was carried out to obtain a DNA fragment of 2.1 Kbp. As shown in Fig. 1, right side, the fragment was cleaved with restriction enzymes SalI and Eco47III, and the product was then ligated with the SalI and SmaI digestion product of pHSG398 (manufactured by Takara Shuzo) using T4 DNA ligase. Competent cells of Escherichia coli JM109 strain were transformed with the reaction mixture. From among the strains resistant to chloramphenicol, a plasmid having the aroG gene was extracted to yield the plasmid pHSG-aroG.

Subsequently, pHSG-aroG was digested with restriction enzymes EcoRI and HindIII, and the resulting DNA fragment bearing the aroG gene was ligated with the EcoRI and HindIII digestion fragment of plasmid pTS1 using T4 DNA ligase. From among the grown strains resistant to ampicillin, the strain in which auxotrophy of L-tyrosine, L-phenylalanine and L-tryptophan disappeared was selected, and a plasmid was extracted therefrom to yield the plasmid pTS-aroG.

Next, after mutation of the plasmid using the hydroxylamine method of Example 1-(1) above, the mutant plasmid was used to transform competent cells of the E. coli AB3257 strain. After ampicillin-resistant strains were isolated, 6 strains which grew in minimum medium supplemented with 10 mM L-phenylalanine were selected. From these strains, plasmids pTS-aroG4, pTS-aroG8, pTS-aroG15, pTS-aroG17, pTS-aroG29 and pTS-aroG40 bearing the aroG gene encoding feedback inhibition-released DS were obtained.

In cells of the AB3257 strain expressing the non-feedback inhibition-released DS, feedback inhibition occurs at a concentration of 10 mM L-phenylalanine in minimum medium. Accordingly, the non-feedback inhibition-suppressed strain fails to synthesize aromatic amino acids such as L-tryptophan and/or L-tyrosine, and therefore, fails to grow.

### (3) Determination of DS enzyme activity

The above plasmids, bearing either mutant aroF (pTS-aroF15 and pTS-aroF33) or mutant aroG (pTS-aroG4, pTS-aroG8, pTS-aroG15, pTS-aroG17, pTS-aroG29 and pTS-aroG40), were used to transform Escherichia coli AB3257 strain having no DS activity. The respective transformants were named AJ 12598 (AB3257/pTS-aroF15), AJ 12599 (AB3257/pTS-aroF33), AJ 12562 (AB3257/pTS-aroG4), AJ 12600 (AB3257/pTS-aroG8), AJ 12563 (AB3257/pTS-aroG15), AJ 12601 (AB3257/pTS-aroG17), AJ 12602 (AB3257/pTS-aroG29) and AJ 12603 (AB3257/pTS-aroG40), respectively. Among them, AJ 12563 and AJ 12603 were deposited as representative strains in the Fermentation Research Institute of the Agency of Industrial Science & Technology of Japan, under the deposit numbers Escherichia coli FERM BP-3567 and FERM BP-3568, respectively. For the purpose of comparison, plasmids hearing wild type genes were also used to transform the E. coli AB3257 strain.

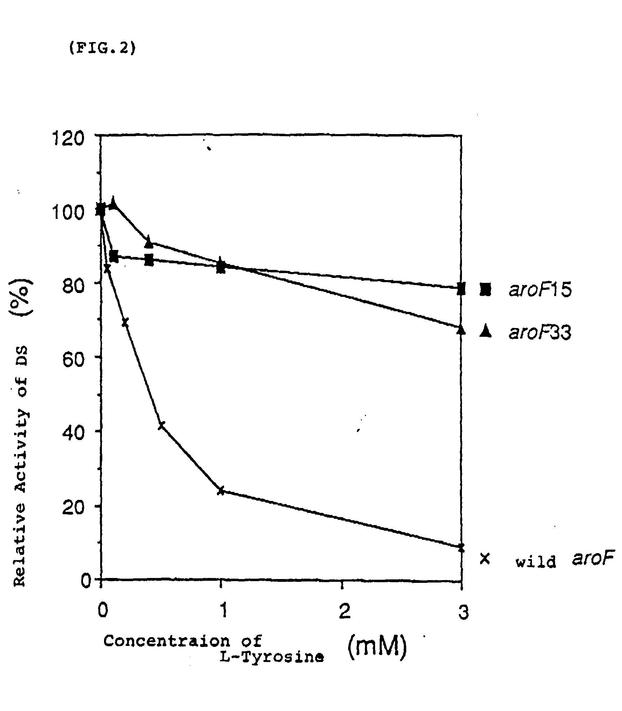

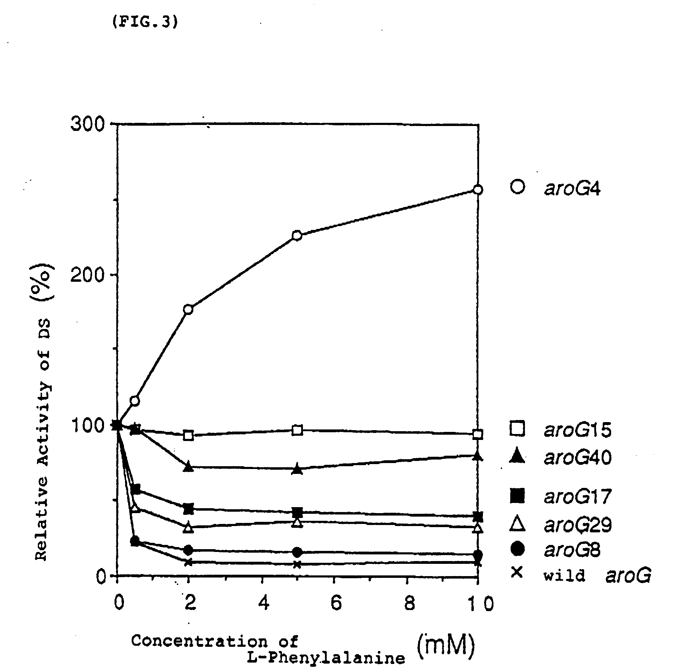

Each of these strains were cultured for 24 hours in a known L-phenylalanine-producing medium [Sugimoto, S. et al., J. Biotechnol., 5, 237 (1988)]. From the culture cells, the crude enzyme solution was prepared by ultrasonic homogenization. The enzyme activity of DS was determined in a conventional manner [Gollub, E. et al., Methods Enzymol., 17, 349], in the presence of L-tyrosine in the case of aroF, and in the presence of L-phenylalanine in the case of aroG. The results presented in Figs. 2 and 3 show that the DS enzyme activity of the wild type transformants (Escherichia coli AB3257/pTS-aroF) is strongly inhibited in the presence of L-tyrosine, whereas the respective mutant transformants are released from feedback inhibition by L-tyrosine. Likewise, in the wild type transformant Escherichia coli AB3257/pTS-aroG, the enzyme activity is strongly inhibited in the presence of L-phenylalanine, whereas in the respective mutant transformants, feedback inhibition by L-phenylalanine is released. Furthermore, the mutant strain AJ 12562 not only releases feedback inhibition by L-phenylalanine, but surprisingly, the DS enzyme activity increases as the concentration of L-phenylalanine increases.

### (4) Detemination of the mutation site of DS in which the feedback inhibition is released

The nuoleotide sequences of the feedback inhibition-released aroF15, aroF33, aroG4, aroG8, aroG15, aroG17, aroG29 and aroG40 were determined in a conventional manner [Molecular Cloning (Second Edition), Cold Spring Harbor Press (1989)]. The specific substitution site on the amino acid sequence and the mutation site on the corresponding nucleotide sequence are shown in Table 1.

These sequences are all novel.

**Table 1**

| Mutant Gene | Substitution Site of Amino Acid | | Corresponding Nucleotide Sequence Change |
|---|---|---|---|
| | Position from N-Terminus | Amino Acid Sequence Change | |
| aroF15 | 147 | Asp → Asn | GAT → AAT |
| aroF33 | 181 | Ser → Phe | TCC → TTC |
| aroG4 | 150 | Pro → Leu | CCA → CTA |
| aroG8 | 202 | Ala → Thr | GCC → ACC |
| aroG15 | 146 | Asp → Asn | GAT → AAT |
| aroG17 | 147 | Met → Ile | ATG → ATA |
| | 332 | Glu → Lys | GAA → AAA |
| aroG29 | 147 | Met → Ile | ATG → ATA |
| aroG40 | 157 | Met → Ile | ATG → ATA |
| | 219 | Ala → Thr | GCG → ACG |

### Example 2: Preparation of a novel gene encoding PD in which the feedback inhibition is released

### (1) Determination of the mutation site of Brevibacterium lactofermentum mutant PD

The nucleotide sequence of the NcoI fragment in plasmid pAJ16 bearing the PD gene of Brevibacterium lactofermentum wild strain was determined by the dideoxy method, using the homology to known Corynebacterium s.p. PD gene [Follettie, M.T. and Sinsky, A.J., J. Bacteriol., 167, 695 (1986)] as an index. The plasmid is harbored on Brevibacterium lactofermentum AJ 12125 (FERM P-7546). The resulting nucleotide sequence (SEQ ID NO:5) and the corresponding amino acid sequence (SEQ ID NO:6) are shown below. The B. lactofermentum PD amino acid sequence is different by only one amino acid residue from that of Corynebacterium s.p.

Next, the nucleotide sequence (SEQ ID NO: 7) of the gene on plasmid pPH14 encoding PD of the phenylalanine-producing strain of Brevibacterium lactofermentum was determined. The sequence shown below was obtained. The plasmid used was the one borne on Brevibacterium lactofermentum AJ 12259 (FERM BP-3565). A comparison of the amino acid sequences was made between the wild-type PD and the feedback inhibition-released PD (SEQ ID NO: 8) and it was found that ²³⁵Ser residue of the wild strain was mutated to a proline residue in the feedback inhibition-released PD.

### (2) Construction of a novel gene encoding a mutant CM-PD of Escherichia coli

Chromosomal DNA was extracted from Escherichia coli K-12 RR1 strain in a conventional manner.

In a separate procedure, four synthetic DNA primers (Sequence Nos. 7-10) (SEQ ID NOS: 9-12) were chemically synthesized in a conventional manner, based on the known nucleotide sequence of the pheA gene [Hudson, G.S. and Davidson, B.E., J. Mol. Biol., 180, 1023 (1984)].

Sequence Nos. 7 and 8 have homologous sequences upstream and downstream from the pheA gene, respectively. Sequence Nos. 9 and 10 are complementary to each other and have almost perfect homology to the sequence around the 330 serine residue (³³⁰Ser), except that one base pair is different, i.e., T (thymine base) is substituted with C (cytosine base). CM-PD of Escherichia coli K-12 has high homology to PD of Brevibacterium lactofermentum. In particular, the 330 serine residue from the N-terminal (³³⁰Ser) of the CM-PD of Escherichia coli K-12 corresponds to the 235 serine residue (²³⁵Ser) of Brevibacterium lactofermentum PD. Sequence Nos. 9 and 10 are synthesized in such a manner that the 330 serine residue becomes a proline residue.

Next, using 1 µg of the chromosomal DNA and either 300 ng of each of the primers of Sequence Nos. 1 and 10, or 300 ng of each of the primers of Sequence Nos. 8 and 9, PCR was carried out to obtain DNA fragments of 1.3 Kbp and 0.5 Kbp, respectively. The PCR temperature cycle of reaction at 94°C for one minute, at 50°C for 2 minutes and at 72°C for 3 minutes was repeated for 20 cycles using a continuous replication reaction device (Thermal Cycler, manufactured by Perkin Elmer Cetus Co.), according to the method of Erlich et al. [PCR Technology, Stockton Press (1989)]. These DNA fragments were subjected to agarose gel electrophoresis and recovered using a standard DNA recovery kit (Gene Clean, manufactured by Funakoshi Co.).

Separately, using these fragments and the primers of Sequence Nos. 7 and 8, PCR reaction was further carried out to obtain a DNA fragment of 1.8 Kbp. After the 1.8 Kbp fragment was digested with BamHI and PstI, a DNA fragment of 1.7 Kbp was recovered by agarose electrophoresis. Subsequently, the 1.7 Kbp fragment was ligated with the BamHI and PstI digestion product of plasmid pHSG398 (manufactured by Takara Shuzo) using T4 DNA ligase. The ligation product was used to transfect Escherichia coli KA197 strain (pheA). Among the strains resistant to chloramphenicol, the strain in which phenylalanine auxotrophy disappeared was selected, and a plasmid was recovered. The plasmid was named pPHAB. Its nucleotide sequence was determined. This plasmid bears the mutant CM-PD enzyme gene in which the 330 serine residue was substituted with a proline residue.

Also using the same methods mentioned above, 330 serine residue from the N-terminal (³³⁰Ser) of the CM-PD of Escherichia coli K-12 was substituted with an aspartic acid residue. Sequence Nos. 11 and 12 were synthesized in such a manner that the 330 serine residue became an aspartic acid residue. In this way, the plasmid pPHAD, which bears the mutant CM-PD enzyme gene in which the 330 serine residue was substituted with an aspartic acid, was obtained.

Also using the same methods mentioned above, amino acid residues downstream from ³³⁰Ser of the CM-PD of Escherichia coli K-12 were deleted. Sequence Nos. 13 and 14 were synthesized in such a manner that the codon of the 330 serine residue became termination codon. In this way, the plasmid pPHATerm, which bears the mutant CM-PD enzyme gene in which the amino acid residues downstream from the 330 serine residue were deleted, was obtained.

### (3) Construction of a tyrA gene-defected W3110 strain of Escherichia coli K-12

Escherichia coli W3110 strain (acquired from National Institute of Heredity) was spread on a plate medium containing streptomycin to obtain a streptomycin-resistant strain. Next, this strain was cultured in a culture medium in combination with Escherichia coli K-12 ME8424 strain (HfrPO45, thi, relA1, tyrA: : Tn10, ung-1, nadB) (acquired from National Institute of Heredity), and the medium was allowed to stand at 37°C for 15 minutes to perform conjugation transfer. Then the medium was applied to a plate medium containing streptomycin, tetracycline and L-tyrosine. The formed colony, i.e., Escherichia coli W3110 (tyrA) strain, was collected.

The plasmid pPHAB, pPHAD, and pPHATerm obtained in Example (2)-2 above were used to transform competent cells of the E. coli W3110 (tyrA) strain. The transformant Escherichia coli K-12 [W3110 (tyrA)/pPHAB) Escherichia coli K-12 [W3110 (tyrA)/pPHAD], and Escherichia coli X-12 [W3110 (tyrA)/pPHATerm] were deposited in the Fermentation Research Institute of the Agency of Industrial Science & Technology of Japan. The deposit numbers are FERM BP-3566, FERM BP-12659, and FERM BP-12662, respectively.

### (4) Measurement of PD enzyme activity

Escherichia coli K-12 W3110 (tyrA)/(pPHAB) strain was cultured at 37°C for 15 hours in L medium and the cells were collected by centrifugation. Then, the cells were washed twice with physiological saline, and suspended in 250 mM Tris-hydrochloride buffer (pH 7.5) containing 0.5 mM dithiothreitol under ice cooling. By ultrasonication (20 KHz) for 30 seconds four times, the crude enzyme solution was prepared.

The PD enzyme activity was determined in a conventional manner [Cotton, R.G.H. and Gibson, F., Meth. in Enzymol., 17, 564 (1970)]. Using the crude enzyme, the enzymatic reaction was carried out at 37°C for 10 minutes in the presence of 50 mM Tris-hydrochloride buffer (pH 8.2) containing 1 mM barium prephenate and 0.5 mM L-tyrosine. Aqueous sodium hydroxide (1 N) was added to terminate the reaction, and the formed phenylpyruvic acid was measured at an extinction wavelength of 320 nm.

Quantitative determination of protein was made using the Protein Assay Kit (manufactured by Bio Rad Co.), according to the protocol of the manufacturer.

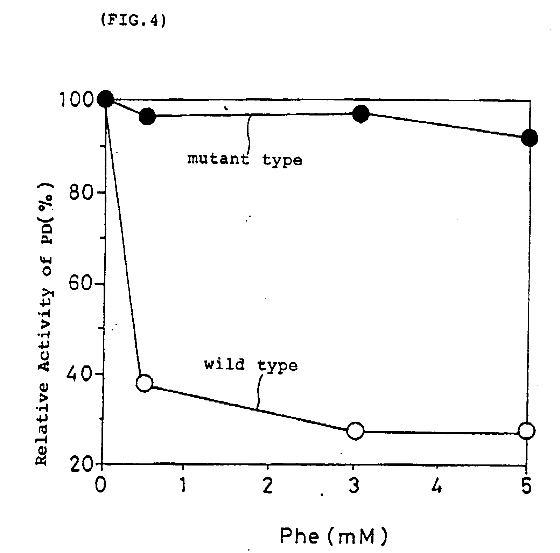

The results presented in Fig. 4 show that the enzyme reaction in strains transformed with the wild CM-PD gene was strongly inhibited in the presence of 0.5 mM L-phenylalanine, whereas strains transformed with a mutant CM-PD gene exhibited almost no inhibition, even in the presence of 5 mM L-phenylalanine.

Further, in the case of the plasmid bearing the wild type enzyme gene, in the absence of L-phenylalanine, the enzyme activity was only 3.5 x 10² U/mg protein. In the case of the mutant CM-PD gene, the activity was 1.5 x 10⁴ U/mg protein. The results show that not only is expression of the mutant type CM-PD enzyme gene released from feedback inhibition by L-phenylalanine in transformants containing the mutant gene, but surprisingly, the amount of enzyme and/or enzyme activity can be increased by roughly two orders of magnitude.

By using the same method above, the PD enzyme activities of Escherichia coli W3110 (tyrA)/(pPHAD) and Escherichia coli W3110 (tyrA)/(pPHATerm) were determined. As the result, the PD enzymes of the both strains were found to be released from feedback inhibition by L-phenylalanine.

### Example 3: Production of L-phenylalanine by fermentation

### (1) Construction of Escherichia coli K-12 bearing a CM-PD gene alone and in combination with a DS gene, in which the feedback inhibition is released

From pTS-aroG4 bearing the feedback inhibition-released DS gene obtained in Example 1, the aroG4 portion was excised with restriction enzymes EcoRI and HindIII. The fragment was inserted into the cleavage site of pBR322 with EcoRI and HindIII to obtain plasmid pBR-aroG4 (having an ampicillin-resistant marker).

In a separate procedure, pPHAB bearing the feedback inhibition-released CM-PD gene obtained in Example 2 was digested with restriction enzymes BamHI and HindIII to excise the fragment containing the CM-PD gene. This fragment was inserted into the cleavage site of pACYC184 with BamHI and HindIII to construct plasmid pACMAB (selection marker was chloramphenicol resistance). The pACAMB plasmid was used to transform competent cells of Escherichia coli K-12 W3110 (tyrA), yielding transformant W3110 (tyrA)/pACMAB.

Furthermore, the two plasmids pACMAB and pBR-aroG4 were used to transform W3110 (tyrA) yielding transformant W3110 (tyrA)/pBR-aroG4,pACMAB. The transformant W3110 (tyrA)/pBR-aroG4,pACMAB was named AJ 12604 strain and deposited in Fermentation Research Institute of the Agency of Industrial Science & Technology of Japan under the deposit number FERM BP-3579.

### (3) Production of L-phenylalanine

The transformant AJ 12604, W3110 (tyrA)/pACMAB, W3110 (tyrA)/pPHAD, W3110 (tyrA)/pPHATerm, and W3110 (tyrA) described above were cultured at 37°C for 24 hours in L-phenylalanine-producing medium (containing 20 g of glucose, 29.4 g of disodium hydrogen phosphate, 6 g of potassium dihydrogen phosphate, 1 g of sodium chloride, 2 g of ammonium chloride, 10 g of sodium citrate, 0.4 g of sodium glutamate, 3 g of magnesium sulfate heptahydrate, 0.23 g of calcium chloride and 2 mg of thiamine hydrochloride in 1 liter of water). The results are shown in Table 2. Quantitative assay was performed by high performance liquid chromatography. An outstanding improvement in the fermentative production of L-phenylalanine using the AJ 12604 strain was obtained.

**Table 2**

| Strain | Amount of L-phenylalanine |
|---|---|
| W3110(tyrA) | 0.1 |
| W3110 (tyrA)/pACMAB | 0.5 |
| W3110 (tyrA)/pPHAD | 0.5 |
| W3110 (tyrA)/pPHATerm | 0.5 |
| AJ 12604 | 3.8 |

### Example 4: Production of L-tryptophan by fermentation

### (1) Construction of a plasmid bearing feedback inhibition-released DS

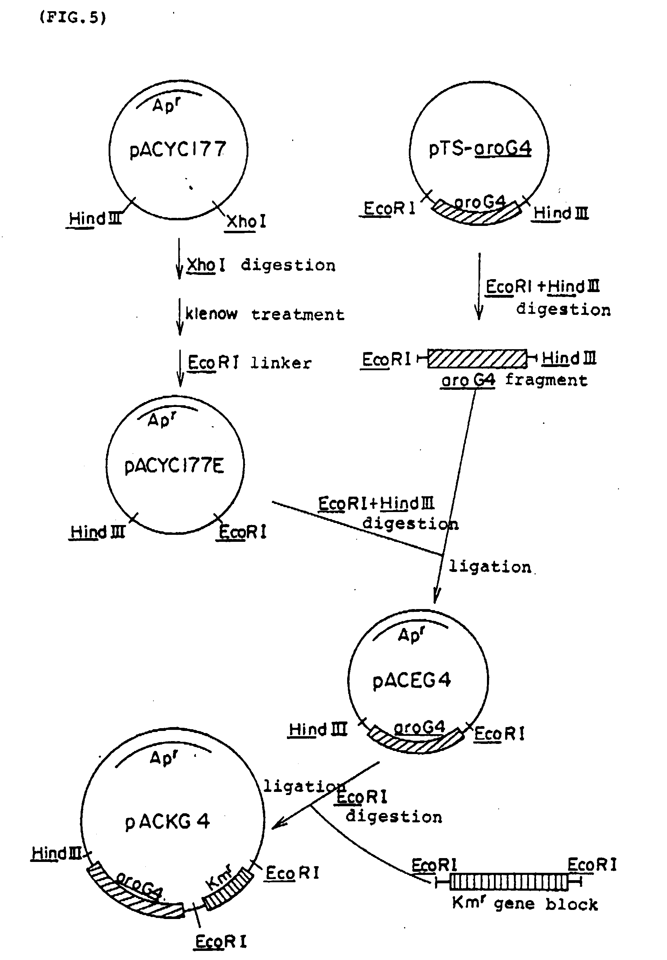

Plasmid pACYC177 (acquired from National Institute of Heredity; ampicillin resistance, 3.6 Kbp) was digested with restriction enzyme XhoI. After the digestion site was made blunt ended by Klenow treatment, an EcoRI linker was ligated therewith using T4 DNA ligase to obtain the plasmid pACYC177E, in which the XhoI site became EcoRI. Next, the plasmid pTS-aroG4 described in Example 1-(2) and 1-(3) above was digested with restriction enzymes EcoRI and HindIII to obtain the fragment containing aroG4. This fragment was ligated with the EcoRI- and HindIII-digested pACYC177E using T4 DNA ligase. Competent cells of the AB3257 strain (described in Example 1) was transformed with the reaction mixture. Among the ampicillin-resistant strains grown, a strain in which auxotrophy for each of L-tyrosine, L-phenylalanine and L-tryptophane disappeared was selected, and a plasmid was extracted. Thus, plasmid pACEG4 (5.1 Kbp) was obtained. An EcoRI-EcoRI fragment containing a gene conferring kanamycin resistance (Kanamycin Gene Block; 1.3 Kbp, manufactured by Pharmacia Fine chemicals) was ligated with the plasmid pACEG4 at the EcoRI site using T4 DNA ligase, thus yielding the plasmid pACKG4 (resistant to ampicillin and kanamycin, 6.4 KbP). The procedure of the construction of pACKG4 is outlined in Fig. 5.

### (2) Construction of Escherichia coli K-12 bearing a feedback inhibition-released DS gene and a tryptophan operon

Competent cells of Escherichia coli K-12 AGX6aroP strain (described in U.S. Patent No. 4,371,614, incorporated herein by reference; deposit number: NRRL B-12264) bearing the plasmid pGX50 harboring a tryptophan operon was transformed with the pACKG4 plasmid described above to obtain Escherichia coli AGX6aroP/pGX50,pACKG4. The genotype of Escherichia coli AGX6aroP strain is tna, trpR+, aroP.

### (3) Production of L-tryptophan

The transformant Escherichia coli AGX6aroP/pGX50,pACKG4 and AGX6aroP/pGX50 described above was cultured at 30°C for 72 hours in L-tryptophan-producing medium (containing 40 g of glucose, 15 g of ammonium sulfate, 1 g of potassium monohydrogen phosphate, 1 g of magnesium sulfate heptahydrate, 0.01 g of ferrous sulfate heptahydrate, 0.01 g of manganese chloride tetrahydrate, 2 g of yeast extract and 40 g of calcium carbonate in 1 liter of water, pH 7). The results are shown in Table 3. Quantitative assay of L-tryptophan was performed by high performance liquid chromatography. An outstanding improvement in the fermentative production of L-tryptophan using the AGXaroP/pGX50,pACKG strain was obtained.

**Table 3**

| Strain | Amount of L-tryptophan Produced (g/l) |
|---|---|
| AGX6aroP/pGX50 | 0.15 |
| AGX6aroP/pGX50,pACKG4 | 0.45 |

Obviously, numerous modifications and variations of the present invention are possible in light of the above teachings. It is therefore to be understood that within the scope of the appended claims, the invention may be practiced otherwise than as specifically described herein.

## Claims

1. A DNA fragment encoding chorismate mutase-prephenate dehydratase from Escherichia coli, in which the serine residue in position 330 is substituted or amino acid residues. downstream from the 330 serine residue are deleted.

2. A DNA fragment encoding prephenate-dehydratase from Coryneform bacterium in which the serine residue in position 235 is substituted.

3. A DNA fragment according to claim 1 or 2, wherein said serine residue is substituted with a proline residue or an aspartic acid residue.

4. A vector, in which the DNA fragment according to any of claims 1 to 3 is operatively linked to regulatory DNA effecting expression of said protein encoding DNA.

5. A microorganism, in which the DNA fragment according to any of claims 1 to 3 is operatively linked to regulatory DNA effecting expression of said protein encoding DNA.

6. A microorganism according to claim 5, which additionally comprises a mutated gene encoding 3-deoxy-D-arabinoheptulonic acid-7-phosphate synthase derived from the genus Escherichia, wherein one or two amino acid residue(s) are substituted or one or more amino acid residue(s) are deleted, and feedback inhibition is released.

7. A microorganism as claimed in claim 6, wherein said 3-deoxy-D-arabinoheptulonic acid-7-phosphate synthase is encoded by aroF.

8. A microorganism as claimed in claim 7, wherein the aspartic acid residue in position 147 or the serine residue in position 181 of said 3-deoxy-D-arabinoheptulonic acid-7-phosphate synthase is substituted with another amino acid residue.

9. A microorganism as claimed in claim 8, wherein said aspartic acid residue is substituted with an asparagine residue.

10. A microorganism as claimed in claime 8, wherein said serine residue is substituted with a phenylalanine residue.

11. A microorganism as claimed in claim 6, wherein said 3-deoxy-D-arabinoheptulonic acid-7-phosphate synthase is encoded by aroG.

12. A microorganism as claimed in claim 11, wherein said one or two amino acid residue(s) of said 3-deoxy-D-arabinoheptulonic acid-7-phosphate synthase being substituted are selected from the group consisting of the aspartic acid residue in position 146, the methionine residue in position 147, the proline residue in position 150, the alanine residue in position 202, and both the methionine residue in position 157, and the alanine residue in position 219.

13. A microorganism as claimed in claim 12, wherein said aspartic acid residue in position 146 is substituted with an asparagine residue, said methionine residue in position 147 is substituted with an isoleucine residue, said proline residue in position 150 is substituted with a leucine residue, said alanine residue in position 202 is substituted with a threonine residue, said methionine residue in position 157 is substituted with an isoleucine residue and said alanine residue in position 219 is substituted with an threonine residue.

14. A process for preparing an aromatic amino acid which comprises culturing a microorganism claimed in any of claims 5 to 13 in a medium, and isolating the aromatic amino acid produced.

15. A process for preparing an aromatic amino acid as claimed in claim 14, wherein said aromatic amino acid is L-phenylalanine or L-tryptophan.

## Patentansprüche

1. DNA-Fragment, welches Chorismatmutase-Prephenatdehydratase aus Escherichia coli codiert, worin der Serinrest in Position 330 substituiert ist oder Aminosäurereste stromabwärts des Serinrestes 330 deletiert sind.

2. DNA-Fragment, welches Prephenatdehydratase aus coryneformen Bakterien codiert, worin der Serinrest in Position 235 substituiert ist.

3. DNA-Fragment gemäß Anspruch 1 oder 2, worin der Serinrest durch einen Prolinrest oder einen Asparaginsäurerest substituiert ist.

4. Vektor, worin das DNA-Fragment gemäß einem der Ansprüche 1 bis 3 funktionsfähig mit regulatorischer DNA verbunden ist, welche die Expression der das Protein codierenden DNA bewirkt.

5. Mikroorganismus, worin das DNA-Fragment gemäß einem der Ansprüche 1 bis 3 funktionsfähig mit regulatorischer DNA verbunden ist, welche die Expression der das Protein codierenden DNA bewirkt.

6. Mikroorganismus gemäß Anspruch 5, der zusätzlich ein mutiertes Gen umfaßt, welches die aus der Gattung Escherichia abgeleitete 3-Deoxy-D-arabinoheptulonsäure-7-phosphatsynthase codiert, worin ein oder zwei Aminosäurereste substituiert sind oder ein oder mehrere Aminosäurereste deletiert sind, und die von der Rückkopplungshemmung befreit ist.

7. Mikroorganismus gemäß Anspruch 6, worin die 3-Deoxy-D-arabinoheptulonsäure-7-phosphatsynthase von aroF codiert wird.

8. Mikroorganismus gemäß Anspruch 7, worin der Asparaginsäurerest in Position 147 oder der Serinrest in Position 181 der 3-Deoxy-D-arabinoheptulonsäure-7-phosphatsynthase durch einen anderen Aminosäurerest substituiert ist.

9. Mikroorganismus gemäß Anspruch 8, worin der Asparaginsäurerest durch einen Asparaginrest substituiert ist.

10. Mikroorganismus gemäß Anspruch 8, worin der Serinrest durch einen Phenylalaninrest substituiert ist.

11. Mikroorganismus gemäß Anspruch 6, worin die 3-Deoxy-D-arabinoheptulonsäure-7-phosphatsynthase von aroG codiert wird.

12. Mikroorganismus gemäß Anspruch 11, worin der eine oder die zwei substituierten Aminosäurereste der 3-Deoxy-D-arabinoheptulonsäure-7-phosphatsynthase aus der Gruppe ausgewählt sind, die aus dem Asparaginsäurerest in Position 146, dem Methioninrest in Position 147, dem Prolinrest in Position 150, dem Alaninrest in Position 202 und den beiden Resten Methioninrest in Position 157 und Alaninrest in Position 219 besteht.

13. Mikroorganismus gemäß Anspruch 12, worin der Asparaginsäurerest in Position 146 durch einen Asparaginrest, der Methioninrest in Position 147 durch einen Isoleucinrest, der Prolinrest in Position 150 durch einen Leucinrest, der Alaninrest in Position 202 durch einen Threoninrest, der Methioninrest in Position 157 durch einen Isoleucinrest und der Alaninrest in Position 219 durch einen Threoninrest substituiert ist.

14. Verfahren zur Herstellung einer aromatischen Aminosäure, welches das Kultivieren eines Mikroorganismus gemäß einem der Ansprüche 5 bis 13 in einem Medium und das Isolieren der hergestellten aromatischen Aminosäure umfaßt.

15. Verfahren zur Herstellung einer aromatischen Aminosäure gemäß Anspruch 14, wobei die aromatische Aminosäure L-Phenylalanin oder L-Tryptophan ist.

## Revendications

1. Fragment d'ADN codant de la chorismate mutase-préphénate déshydratase de Escherichia coli, dans lequel le résidu sérine à la position 330 est substitué ou les résidus acides aminés en aval du résidu sérine 330 sont délétés.

2. Fragment d'ADN codant de la préphénate-déshydratase de Corynébactérium dans lequel le résidu sérine à la position 235 est substitué.

3. Fragment d'ADN selon la revendication 1 ou 2, dans lequel ledit résidu sérine est substitué par un résidu proline ou un résidu acide aspartique.

4. Vecteur dans lequel le fragment d'ADN selon l'une quelconque des revendications 1 à 3 est lié fonctionnellement à un ADN de régulation effectuant l'expression dudit ADN codant les protéines.

5. Micro-organisme dans lequel le fragment d'ADN selon l'une quelconque des revendications 1 à 3 est fonctionnellement lié à un ADN de régulation effectuant l'expression dudit ADN codant les protéines.

6. Micro-organisme selon la revendication 5, lequel comprend additionnellement un gène muté codant la 7-phosphate d'acide 3-désoxy-D-arabinoheptulonique-synthase provenant du genre Escherichia, dans lequel un ou deux résidus acides aminés sont substitués ou un ou plusieurs résidus acides aminés sont délétés et la rétro-inhibition est supprimée.

7. Micro-organisme selon la revendication 6, dans lequel ladite 7-phosphate d'acide 3-désoxy-D-arabinoheptulonique-synthase est codée par aroF.

8. Micro-organisme selon la revendication 7, dans lequel le résidu acide aspartique à la position 147 ou le résidu sérine à la position 181 de ladite 7-phosphate d'acide 3-désoxy-D-arabinoheptulonique-synthase est substitué par un autre résidu acide aminé.

9. Micro-organisme selon la revendication 8, dans lequel ledit résidu acide aspartique est substitué par un résidu asparagine.

10. Micro-organisme selon la revendication 8, dans lequel ledit résidu sérine est substitué par un résidu phénylalanine.

11. Micro-organisme selon la revendication 6, dans lequel ladite 7-phosphate d'acide 3-désoxy-D-arabinoheptulonique-synthase est codée par aroG.

12. Micro-organisme selon la revendication 11, dans lequel lesdits un ou deux résidus acides aminés de ladite 7-phosphate d'acide 3-désoxy-D-arabinoheptulonique-synthase substitués sont sélectionnés dans le groupe constitué par le résidu acide aspartique à la position 146, le résidu méthionine à la position 147, le résidu proline à la position 150, le résidu alanine à la position 202, le résidu méthionine à la position 157 et le résidu alanine à la position 219.

13. Micro-organisme selon la revendication 12, dans lequel ledit résidu acide aspartique à la position 146 est substitué par un résidu asparagine, ledit résidu méthionine à la position 147 est substitué par un résidu isoleucine, ledit résidu proline à la position 150 est substitué par un résidu leucine, ledit résidu alanin à la position 202 est substitué par un résidu thréonine, ledit résidu méthionine à la position 157 est substitué par un résidu isoleucine et ledit résidu alanine à la position 219 est substitué par un résidu thréonine.

14. Procédé de préparation d'un acide aminé aromatique, lequel comprend la culture d'un micro-organisme selon l'une quelconque des revendications 5 à 13 dans un milieu et l'isolement de l'acide aminé aromatique produit.

15. Procédé de préparation d'un acide aminé aromatique selon la revendication 14, dans lequel ledit acide aminé aromatique est la L-phénylalanine ou le L-tryptophane.


### SEQUENCE LISTING

### (1) GENERAL INFORMATION:

(i) APPLICANT: Ajinomoto Co., Ltd.
(ii) TITLE OF INVENTION: GENES ENCODING FEEDBACK INHIBITION-RELEASED ENZYMES, PLASMIDS CONTAINING THE GENES, AND MICROORGANISMS TRANSFORMED WITH THE PLASMIDS USEFUL IN PROCESSES FOR PREPARING AROMATIC AMINO ACIDS BY FERMENTAT
(iii) NUMBER OF SEQUENCES: 12

### (2) INFORMATION FOR SEQ ID NO:1:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 MC1061
(xi) SEQUENCE DESCRIPTION: SEQ ID NO:1:
   GCTAACCAGT AAAGCCAACA 20

### (2) INFORMATION FOR SEQ ID NO:2:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 MC1061
(xi) SEQUENCE DESCRIPTION: SEQ ID NO:2:
   CCCACTTCAG CAACCAGTTC 20

### (2) INFORMATION FOR SEQ ID.NO:3:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 MC1061
(xi) SEQUENCE DESCRIPTION: SEQ ID NO:3:
   GTATTTACCC CGTTATTGTC 20

### (2) INFORMATION FOR SEQ ID No:4:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 MC1061
(xi) SEQUENCE DESCRIPTION: SEQ ID NO:4:
   ACTCCGCCGG AAGTGACTAA 20

### (2) INFORMATION FOR SEQ ID NO:5:

(i) SEQUENCE CHARACTERISTICS:
   (A) LENGTH: 948 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Brevibacterium lactofermentum
   (B) STRAIN: AJ 12125
(ix) FEATURE:
   (A) NAME/KEY: CDS
   (B) LOCATION: 1..945
(xi) SEQUENCE DESCRIPTION: SEQ ID NO:5:
   ATG AGC CAC CCA CCA ATT GTT GTG GCC TAT TTG GGG CCT GCC GGA ACC        48
   Met Ser Asp Ala Pro Ile Val Val Ala Tyr Leu Gly Pro Ala Gly Thr
     1               5                  10                  15
   TTC ACC GAA GAA GCC CTC TAC AAA TTT GCC GAC GCC GGC GTA TTC GGC        96
   Phe Thr Glu Glu Ala Leu Tyr Lys Phe Ala Asp Ala Gly Val Phe Gly
                20                  25                  30
   GAC GGT GAG ATC GAG CAG CTA CCA GCC AAA TCG CCA CAA GAA GCT GTC       144
   Asp Gly Glu Ile Glu Gln Leu Pro Ala Lys Ser Pro Gln Glu Ala Val
            35                  40                  45
   GAC GCG GTC CGC CAC GGC ACC GCC CAG TTC GCG GTG GTC GCC ATC GAA       192
   Asp Ala Val Arg His Gly Thr Ala Gln Phe Ala Val Val Ala Ile Glu
        50                  55                  60
   AAC TTC GTC GAC GGC CCC GTC ACC CCC ACC TTC GAC GCC CTT GAC CAG       240
   Asn Phe Val Asp Gly Pro Val Thr Pro Thr Phe Asp Ala Leu Asp Gln
    65                  70                  75                  80
   GGC TCC AAC GTG CAA ATC ATC GCC GAA GAA GAA CTC GAT ATT GCC TTT       288
   Gly Ser Asn Val Gln Ile Ile Ala Glu Glu Glu Leu Asp Ile Ala Phe
                    85                  90                  95
   TCC ATC ATG GTC CGG CCA GGG ACT TCG CTT GCC GAC GTC AAA ACC CTC       336
   Ser Ile Met Val Arg Pro Gly Thr Ser Leu Ala Asp Val Lys Thr Leu
               100                 105                 110
   GCC ACC CAC CCG GTT GGG TAC CAA CAA GTG AAA AAC TGG ATG GCA ACC       384
   Ala Thr His Pro Val Gly Tyr Gln Gln Val Lys Asn Trp Met Ala Thr
           115                 120                 125
   ACC ATT CCG GAC GCC ATG TAT CTT TCA GCA AGC TCC AAC GGC GCC GGC       432
   Thr Ile Pro Asp Ala Met Tyr Leu Ser Ala Ser Ser Asn Gly Ala Gly
       130                 135                 140
   GCA CAA ATG GTT GCC GAA GGA ACC GCC GAC GCA GCC GCA GCG CCC TCC       480
   Ala Gln Met Val Ala Glu Gly Thr Ala Asp Ala Ala Ala Ala Pro Ser
   145                 150                 155                 160
   CGC GCA GCC GAA CTC TTC GGA CTG GAA CGC CTT GTT GAT GAT GTC GCC       528
   Arg Ala Ala Glu Leu Phe Gly Leu Glu Arg Leu Val Asp Asp Val Ala
                   165                 170                 175
   GAC GTC CGC GGC GCC CGC ACC CGC TTC GTT GCA GTC CAA GCC CAA GCA       576
   Asp Val Arg Gly Ala Arg Thr Arg Phe Val Ala Val Gln Ala Gln Ala
               180                 185                 190
   GCC GTT TCC GAA CCG ACC GGC CAC GAC CGC ACC TCC GTC ATT TTC TCC       624
   Ala Val Ser Glu Pro Thr Gly His Asp Arg Thr Ser Val Ile Phe Ser
           195                 200                 205
   CTA CCG AAT GTG CCA GGC AGC CTC GTG CGC GCC CTC AAC GAA TTC GCC      672
   Leu Pro Asn Val Pro Gly Ser Leu Val Arg Ala Leu Asn Glu Phe Ala
       210                 215                 220
   ATC CGT GGC GTC GAC CTC ACC CGC ATC GAA TCC CGC CCC ACC CGC AAA      720
   Ile Arg Gly Val Asp Leu Thr Arg Ile Glu Ser Arg Pro Thr Arg Lys
   225                 230                 235                 240
   GTC TTC GGA ACC TAC CGC TTC CAC CTG GAC ATA TCC GGA CAT ATC CGC      768
   Val Phe Gly Thr Tyr Arg Phe His Leu Asp Ile Ser Gly His Ile Arg
                   245                 250                 255
   GAC ATC CCC GTC GCC GAA GCC CTC CGC GCA CTC CAC CTC CAA GCC GAA      816
   Asp Ile Pro Val Ala Glu Ala Leu Arg Ala Leu His Leu Gln Ala Glu
               260                 265                 270
   GAA CTC GTA TTC GTC GGT TCC TGG CCC TCC AAC CGT GCA GAA GAC AGC      864
   Glu Leu Val Phe Val Gly Ser Trp Pro Ser Asn Arg Ala Glu Asp Ser
           275                 280                 285
   ACG CCC CAA ACC GAC CAA CTA GCT AAC GTA CAC AAG GCG GAC GAA TGG      912
   Thr Pro Gln Thr Asp Gln Leu Ala Asn Val His Lys Ala Asp Glu Trp
       290                 295                 300
   GTT CGC GCA GCA AGC GAA GGA AGG AAA CTT AAC TAG                      948
   Val Arg Ala Ala Ser Glu Gly Arg Lys Leu Asn
   305                 310                 315

### (2) INFORMATION FOR SEQ ID NO:6:

(i) SEQUENCE CHARACTERISTICS:
   (A) LENGTH: 315 amino acids
   (B) TYPE: amino acid
   (D) TOPOLOGY: linear
(ii) MOLECULE TYPE: protein
(xi) SEQUENCE DESCRIPTION: SEQ ID NO:6:
   Met Ser Asp Ala Pro Ile Val Val Ala Tyr Leu Gly Pro Ala Gly Thr
   1               5                   10                  15
   Phe Thr Glu Glu Ala Leu Tyr Lys Phe Ala Asp Ala Gly Val Phe Gly
               20                  25                  30
   Asp Gly Glu Ile Glu Gln Leu Pro Ala Lys Ser Pro Gln Glu Ala Val
           35                  40                  45
   Asp Ala Val Arg His Gly Thr Ala Gln Phe Ala Val Val Ala Ile Glu
       50                  55                  60
   Asn Phe Val Asp Gly Pro Val Thr Pro Thr Phe Asp Ala Leu Asp Gln
   65                  70                  75                  80
   Gly Ser Asn Val Gln Ile Ile Ala Glu Glu Glu Leu Asp Ile Ala Phe
                   85                  90                  95
   Ser Ile Met Val Arg Pro Gly Thr Ser Leu Ala Asp Val Lys Thr Leu
               100                 105                 110
   Ala Thr His Pro Val Gly Tyr Gln Gln Val Lys Asn Trp Met Ala Thr
           115                 120                 125
   Thr Ile Pro Asp Ala Met Tyr Leu Ser Ala Ser Ser Asn Gly Ala Gly
       130                 135                 140
   Ala Gln Met Val Ala Glu Gly Thr Ala Asp Ala Ala Ala Ala Pro Ser
   145                 150                 155                 160
   Arg Ala Ala Glu Leu Phe Gly Leu Glu Arg Leu Val Asp Asp Val Ala
                   165                 170                 175
   Asp Val Arg Gly Ala Arg Thr Arg Phe Val Ala Val Gln Ala Gln Ala
               180                 185                 190
   Ala Val Ser Glu Pro Thr Gly His Asp Arg Thr Ser Val Ile Phe Ser
           195                 200                 205
   Leu Pro Asn Val Pro Gly Ser Leu Val Arg Ala Leu Asn Glu Phe Ala
       210                 215                 220
   Ile Arg Gly Val Asp Leu Thr Arg Ile Glu Ser Arg Pro Thr Arg Lys
   225                 230                 235                 240
   Val Phe Gly Thr Tyr Arg Phe His Leu Asp Ile Ser Gly His Ile Arg
                   245                 250                 255
   Asp Ile Pro Val Ala Glu Ala Leu Arg Ala Leu His Leu Gln Ala Glu
               260                 265                 270
   Glu Leu Val Phe Val Gly Ser Trp Pro Ser Asn Arg Ala Glu Asp Ser
           275                 280                 285
   Thr Pro Gln Thr Asp Gln Leu Ala Asn Val His Lys Ala Asp Glu Trp
       290                 295                 300
   Val Arg Ala Ala Ser Glu Gly Arg Lys Leu Asn
   305                 310                 315

### (2) INFORMATION FOR SEQ ID NO:7:

(i) SEQUENCE CHARACTERISTICS:
   (A) LENGTH: 948 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Brevibacterium lactofermentum
   (B) STRAIN: AJ 12259
(xi) SEQUENCE DESCRIPTION: SEQ ID NO:7:
   ATGAGCGACGCACCAATTGTTGTGGCCTATTTGGGGCCTGCCGGAACCTTCACCGAAGAA     60
   GCCCTCTACAAATTTGCCGACGCCGGCGTATTCGGCGACGGTGAGATCGAGCAGCTACCA    120
   GCCAAATCGCCACAAGAAGCTGTCGACGCGGTCCGCCACGGCACCGCCCAGTTCGCGGTG    180
   GTCGCCATCGAAAACTTCGTCGACGGCCCCGTCACCCCCACCTTCGACGCCCTTGACCAG    240
   GGCTCCAACGTGCAAATCATCGCCGAAGAAGAACTCGATATTGCCTTTTCCATCATGGTC    300
   CGGCCAGGGACTTCGCTTGCCGACGTCAAAACCCTCGCCACCCACCCGGTTGGGTACCAA    360
   CAAGTGAAAAACTGGATGGCAACCACCATTCCGGACGCCATGTATCTTTCAGCAAGCTCC    420
   AACGGCGCCGGCGCACAAATGGTTGCCGAAGGAACCGCCGACGCAGCCGCAGCGCCCTCC    480
   CGCGCAGCCGAACTCTTCGGACTGGAACGCCTTGTTGATGATGTCGCCGACGTCCGCGGC    540
   GCCCGCACCCGCTTCGTTGCAGTCCAAGCCCAAGCAGCCGTTTCCGAACCGACCGGCCAC    600
   GACCGCACCTCCGTCATTTTCTCCCTACCGAATGTGCCAGGCAGCCTCGTGCGCGCCCTC    660
   AACGAATTCGCCATCCGTGGCGTCGACCTCACCCGCATCGAATCCCGCCCCACCCGCAAA    720
   GTCTTCGGAACCTACCGCTTCCACCTGGACATATCCGGACATATCCGCGACATCCCCGTC    780
   GCCGAAGCCCTCCGCGCACTCCACCTCCAAGCCGAAGAACTCGTATTCGTCGGTTCCTGG    840
   CCCTCCAACCGTGCAGAAGACAGCACGCCCCAAACCGACCAACTAGCTAACGTACACAAG    900
   GCGGACGAATGGGTTCGCGCAGCAAGCGAAGGAAGGAAACTTAACTAG                948

### (2) INFORMATION FOR SEQ ID NO:8:

(i) SEQUENCE CHARACTERISTICS:
   (A) LENGTH: 315 amino acids
   (B) TYPE: amino acid
   (D) TOPOLOGY: linear
(ii) MOLECULE TYPE: protein
(xi) SEQUENCE DESCRIPTION: SEQ ID NO:8:
   Met Ser Asp Ala Pro Ile Val Val Ala Tyr Leu Gly Pro Ala Gly Thr
   1               5                   10                  15
   Phe Thr Glu Glu Ala Leu Tyr Lys Phe Ala Asp Ala Gly Val Phe Gly
               20                  25                  30
   Asp Gly Glu Ile Glu Gln Leu Pro Ala Lys Ser Pro Gln Glu Ala Val
           35                  40                  45
   Asp Ala Val Arg His Gly Thr Ala Gln Phe Ala Val Val Ala Ile Glu
       50                  55                  60
   Asn Phe Val Asp Gly Pro Val Thr Pro Thr Phe Asp Ala Leu Asp Gln
   65                  70                  75                  80
   Gly Ser Asn Val Gln Ile Ile Ala Glu Glu Glu Leu Asp Ile Ala Phe
                   85                  90                  95
   Ser Ile Met Val Arg Pro Gly Thr Ser Leu Ala Asp Val Lys Thr Leu
               100                 105                 110
   Ala Thr His Pro Val Gly Tyr Gln Gln Val Lys Asn Trp Met Ala Thr
           115                 120                 125
   Thr Ile Pro Asp Ala Met Tyr Leu Ser Ala Ser Ser Asn Gly Ala Gly
       130                 135                 140
   Ala Gln Met Val Ala Glu Gly Thr Ala Asp Ala Ala Ala Ala Pro Ser
   145                 150                 155                 160
   Arg Ala Ala Glu Leu Phe Gly Leu Glu Arg Leu Val Asp Asp Val Ala
                   165                 170                 175
   Asp Val Arg Gly Ala Arg Thr Arg Phe Val Ala Val Gln Ala Gln Ala
               180                 185                 190
   Ala Val Ser Glu Pro Thr Gly His Asp Arg Thr Ser Val Ile Phe Ser
           195                 200                 205
   Leu Pro Asn Val Pro Gly Ser Leu Val Arg Ala Leu Asn Glu Phe Ala
       210                 215                 220
   Ile Arg Gly Val Asp Leu Thr Arg Ile Glu Pro Arg Pro Thr Arg Lys
   225                 230                 235                 240
   Val Phe Gly Thr Tyr Arg Phe His Leu Asp Ile Ser Gly His Ile Arg
   245 250 255
   Asp Ile Pro Val Ala Glu Ala Leu Arg Ala Leu His Leu Gln Ala Glu
   260 265 270
   Glu Leu Val Phe Val Gly Ser Trp Pro Ser Asn Arg Ala Glu Asp Ser
   275 280 285
   Thr Pro Gln Thr Asp Gln Leu Ala Asn Val His Lys Ala Asp Glu Trp
   290 295 300
   Val Arg Ala Ala Ser Glu Gly Arg Lys Leu Asn
   305 310 315

### (2) INFORMATION FOR SEQ ID NO:9:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 RR1
(xi) SEQUENCE DESCRIPTION: SEQ ID NO:9:
   TCAACAAGCT GGAACGGACG 20

### (2) INFORMATION FOR SEQ ID NO:10:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(vi) ORIGINAL SOURCE:
   (A) ORGANISM: Escherichia coli
   (B) STRAIN: K-12 RR1
(xi) SEQUENCE DESCRIPTION: SEQ ID NO: 10:
   CGCCGATTTA CCGCCTTGAG 20

### (2) INFORMATION FOR SEQ ID NO:11:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(xi) SEQUENCE DESCRIPTION: SEQ ID NO:11:
   CCGTCTGGAA CCACGCCCGA T 21

### (2) INFORMATION FOR SEQ ID NO:12:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: NO
(xi) SEQUENCE DESCRIPTION: SEQ ID NO:12:
   ATCGGGCGTG ATTCCAGACG G 21